# EUROPEAN PATENT APPLICATION

(11) **EP 4 835 091 A1**
(43) Date of publication of application: **04.11.2026**
(21) Application number: 24913111.1
(22) Date of filing: 27.12.2024
(51) Int. Cl.: C12P 21/08, A61K 39/395, C12N 5/10, C12N 15/13, C12N 15/63

(54) **METHOD FOR PRODUCING ANTIBODIES**

(30) Priority: 27.12.2023 WO PCT/JP2023/046988
(71) Applicant: CHUGAI SEIYAKU KABUSHIKI KAISHA, Tokyo 115-8543 (JP)
(72) Inventor: ISHIKAWA, Yuya, Tokyo 115-8543 (JP)
(74) Representative: Vossius & Partner Patentanwälte Rechtsanwälte mbB
(86) International application number: PCT/JP2024/046411
(87) International publication number: WO 2025/143225

(57) **Abstract**

The present invention provides a method for producing a recombinant antibody efficiently and cost-effectively. Specifically, the present invention provides a method for producing an antibody or a fragment thereof, the method being characterized by using a cell introduced with a vector containing 4 copies of DNA encoding an L chain of an antibody or a fragment thereof and 2 copies of DNA encoding an H chain of the antibody or a fragment thereof. The present invention also provides a recombinant vector including 4 copies of DNA encoding an L chain of an antibody or a fragment thereof and 2 copies of DNA encoding an H chain of the antibody or a fragment thereof, and a transformed cell introduced with such a vector.

## Description

### TECHNICAL FIELD

The present invention relates to a method for producing an antibody.

### BACKGROUND ART

The use of animal cells to produce recombinant antibodies useful as pharmaceuticals through genetic recombination technology enables complex post-translational modifications and folding that cannot be achieved in prokaryotic cells. Thus, animal cells have been widely used as host cells for recombinant antibody production.

In recent years, many biopharmaceuticals such as antibodies and biologically active proteins have been produced. In particular, for antibody pharmaceuticals, a single dose is typically in the mg range, requiring a considerable amount of antibodies as the active ingredient. The techniques that efficiently produces recombinant antibodies in animal cells will lead to the reduction of costs for antibody pharmaceuticals, ensuring a stable supply to patients.

Thus, there are demands for recombinant-antibody production methods with higher production efficiency.

For example, it is important to develop expression systems for efficiently producing recombinant antibodies in animal cells.

EP2439269 (PTL 1) discloses the use of an expression vector containing a drug selection marker gene expression cassette introduced with an mRNA destabilizing sequence, and a gene expression stabilizing element to establish transformed cells that express the heavy chain and/or light chain polypeptide genes of an antibody at high levels.

When generating host cells for producing a recombinant antibody, typically one copy of DNA encoding the H chain and one copy of DNA encoding the L chain of the antibody are introduced into the host cells (NPL 1 and 2).

However, WO2009/051108 (PTL 2) discloses a high-yield antibody production method using transformed cells that contain a higher copy number of DNA encoding the L chain of an antibody than DNA encoding the H chain of the antibody. In WO2009/051108, a plasmid containing one copy of DNA encoding the H chain of a desired recombinant antibody and two copies of DNA encoding the L chain of the antibody is introduced into cells.

The H-chain polypeptide and L-chain polypeptide that constitute an antibody molecule assemble with the support of BiP (immunoglobulin heavy chain binding protein), and subsequently undergo folding to form the full antibody structure. This assembly process is dependent on the L-chain polypeptide (NPL 3). Thus, it is considered that the assembly of the H chain polypeptide and L chain polypeptide is promoted and the production is increased by increasing the ratio of L chain gene numbers and the ratio of L chain polypeptides.

There is a disclosed method for producing antibodies, which includes performing transient expression or stable expression using Chinese Hamster ovary (CHO) cells containing a higher copy number of exogenous DNA encoding the L chain of an antibody than DNA encoding the H chain of the antibody (NPL 4). In this antibody production method, the exogenous DNA encoding the L chain of the antibody and the exogenous DNA encoding the H chain of the antibody are introduced into the cells using separate vectors.

However, particularly in the case of stable expression transformed cells (stable transformant) expressing recombinant antibodies in a stable expression system, the expression of the H chain is suggested to be more important because the expression of the H chain decreases compared to that in a transient expression system (NPL 4). Thus, in the case of a stable expression system, it is unclear how to control the expression ratio of the H chain and the L chain to increase the production of antibodies.

### CITATION LIST

### PATENT LITERATURE

PTL 1: EP2439269
PTL 2: International Publication No. WO2009/051108

### NON PATENT LITERATURE

NPL 1: Reff ME, Carner K, Chambers KS, Chinn PC, Leonard JE, Raab R et al. Depletion of B cells in vivo by a chimeric mouse human monoclonal antibody to CD20. Blood. 1994 Jan 15; 83(2):435-45
NPL 2: Presta LG, Chen H, O'Connor SJ, Chisholm V, Meng YG, Krummen L, et al. Humanization of an anti-vascular endothelial growth factor monoclonal antibody for the therapy of solid tumors and other disorders. Cancer Res. 1997 Oct 15; 57(20):4593-9
NPL 3: Young-Kwang Lee, Joseph W. Brewer, Rachel Hellman, and Linda M. Hendershot. BiP and immunoglobulin light chain cooperate to control the folding of heavy chain and ensure the fidelity of immunoglobulin assembly. Molecular Biology of the Cell, 1999, 10, 2209
NPL 4: Stefan Schlatter, et al. On the Optimal Ratio of Heavy to Light Chain Genes for Efficient Recombinant Antibody Production by CHO Cells. Biotechnol. Prog., 2005, 21, 122

### SUMMARY OF INVENTION

### TECHNICAL PROBLEM

An object of the present invention is to provide a method for producing antibodies at high yield.

### SOLUTION TO PROBLEM

The present inventors have created CHO cells introduced with an expression vector containing 4 copies of DNA encoding the L chain of an antibody and 2 copies of DNA encoding the H chain of the antibody, and have found that the cells can provide a cell line with superior antibody productivity compared to 1) cells introduced with a vector containing 2 copies of the L chain and 1 copy of the H chain, 2) cells introduced with a vector containing 6 copies of the L chain and 3 copies of the H chain, and 3) cells introduced with a vector containing 8 copies of the L chain and 4 copies of the H chain, thereby completing the present invention.

The summary of the present invention is as follows.
(1) A method for producing an antibody or a fragment thereof, the method comprising using a cell introduced with a vector containing 4 copies of DNA encoding an L chain of an antibody or a fragment thereof and 2 copies of DNA encoding an H chain of the antibody or a fragment thereof.
(2) The method according to claim 1, wherein the antibody or a fragment thereof is produced by constructing a recombinant vector containing 4 copies of DNA encoding an L chain of an antibody or a fragment thereof and 2 copies of DNA encoding an H chain of the antibody or a fragment thereof, introducing the vector into a cell, and culturing the transformed cell introduced with the vector to produce the antibody or a fragment thereof.
(3) The method according to (1) or (2), wherein the cell is an animal cell.
(4) The method according to any of (1) to (3), wherein the animal cell is a Chinese hamster ovary cell.
(5) The method according to any of (1) to (4), wherein the antibody is a chimeric antibody, a humanized antibody, or a human antibody.
(6) The method according to any of (1) to (5), wherein the antibody is a switch antibody, a recycling antibody, or a sweeping antibody.
(7) The method according to any of (1) to (6), wherein the antibody is selected from the group consisting of an anti-CD137 antibody, an anti-latent TGF-β1 antibody, an anti-latent myostatin antibody, an anti-complement (C1s) antibody, an anti-IL-8 antibody, an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody, and preferably the antibody is an anti-CD137 agonist switch antibody STA551, an anti-latent TGF-β1 monoclonal antibody SOF10/RG6440, an anti-latent myostatin sweeping antibody GYM329/RG6237, an anti-complement (C1s) antibody RAY121, an antibody S12pre, or an anti-IL-8 recycling antibody AMY109.
(8) The method according to any of (1) to (7), wherein the cell stably expresses the antibody or a fragment thereof.
(9) A recombinant vector comprising 4 copies of DNA encoding an L chain of an antibody or a fragment thereof and 2 copies of DNA encoding an H chain of the antibody or a fragment thereof.
(10) A cell introduced with the vector according to (9).
(11) A cultured cell introduced with a vector that contains 4 copies of exogenous DNA encoding an L chain of an antibody or a fragment thereof and 2 copies of exogenous DNA encoding an H chain of the antibody or a fragment thereof in the same vector.
(12) The cell according to (10) or (11), which stably expresses the antibody or a fragment thereof.
(13) The vector according to (8) or the cell according to any of (9) to (12), wherein the antibody is selected from the group consisting of an anti-CD137 antibody, an anti-latent TGF-β1 antibody, an anti-latent myostatin antibody, an anti-complement (C1s) antibody, an anti-IL-8 antibody, an anti-IL-6 receptor antibody, an anti-IL-6 antibody, an anti-glypican 3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody, and preferably the antibody is an anti-CD137 agonist switch antibody STA551, an anti-latent TGF-β1 monoclonal antibody SOF10/RG6440, an anti-latent myostatin sweeping antibody GYM329/RG6237, an anti-complement (C1s) antibody RAY121, an antibody S12pre, or an anti-IL-8 recycling antibody AMY109.
(14) The method according to any of (1) to (8), the vector according to (9), or the cell according to any of (10) to (13), wherein the antibody is a humanized anti-CD137 antibody (e.g., STA551).
(15) A method for producing a pharmaceutical containing an antibody or a fragment thereof, wherein the antibody or a fragment thereof is produced by the method according to any of (1) to (8), and the obtained antibody is mixed and formulated with a pharmaceutically acceptable carrier or additive to prepare the pharmaceutical.

### ADVANTAGEOUS EFFECTS OF INVENTION

In the present invention, setting the copy number of cDNA for the L chain and H chain of an antibody contained in an expression vector to a specific number (4 copies for the L chain and 2 copies for the H chain) enables obtaining a transformed cell with a high production of the desired recombinant antibody. The present invention makes it possible to provide a novel method for producing recombinant antibodies with high production efficiency. Furthermore, since the plasmid is smaller in size compared to the H3L6 plasmid composed of 3 copies of the H chain and 6 copies of the L chain, and the H4L8 plasmid composed of 4 copies of the H chain and 8 copies of the L chain, it is expected that the plasmid is easy to construct, the introduction efficiency of plasmid into cells is high, and the plasmid is stably maintained after being introduced into the host cell genome.

### BRIEF DESCRIPTION OF DRAWINGS

Fig. 1 shows an H2L4 plasmid composed of 2 copies of the H chain and 4 copies of the L chain of a humanized anti-CD137 antibody gene.
Fig. 2 shows an H3L6 plasmid composed of 3 copies of the H chain and 6 copies of the L chain of the humanized anti-CD137 antibody gene.
Fig. 3 shows an H4L8 plasmid composed of 4 copies of the H chain and 8 copies of the L chain of the humanized anti-CD137 antibody gene.
Fig. 4 is a graph comparing the titers of H2L4 plasmid-introduced clonal cell lines (median), H3L6 plasmid-introduced clonal cell lines (average), and H4L8-introduced clonal cell lines (median).
Fig. 5 shows an H1L2 plasmid composed of 1 copy of the H chain and 2 copies of the L chain of the humanized anti-CD137 antibody gene.
Fig. 6 is a graph comparing the titers of H1L2 plasmid-introduced clonal cell lines (median) and H2L4 plasmid-introduced clonal cell lines (median).

### DESCRIPTION OF EMBODIMENTS

Hereinafter, the embodiments of the present invention will be described in detail.

The present invention provides a method for producing an antibody or a fragment thereof, comprising producing the antibody or a fragment thereof by using a cell introduced with a vector containing 4 copies of exogenous DNA encoding an L chain of an antibody or a fragment thereof and 2 copies of exogenous DNA encoding an H chain of the antibody or a fragment thereof. In the production method of the present invention, 4 copies of DNA encoding the L chain or a fragment thereof and 2 copies of DNA encoding the H chain or a fragment thereof are all contained in the same vector.

The present invention also provides a recombinant vector comprising 4 copies of exogenous DNA encoding an L chain of an antibody or a fragment thereof and 2 copies of exogenous DNA encoding an H chain of the antibody or a fragment thereof.

The present invention also provides a cell introduced with a vector comprising 4 copies of exogenous DNA encoding an L chain of an antibody or a fragment thereof and 2 copies of exogenous DNA encoding an H chain of the antibody or a fragment thereof.

The present invention also provides a cultured cell introduced with a vector comprising 4 copies of exogenous DNA encoding an L chain of an antibody or a fragment thereof and 2 copies of exogenous DNA encoding an H chain of the antibody or a fragment thereof.

In the present invention, it is preferred that the aforementioned cell or cultured cell is a transformed cell introduced with a vector containing 4 copies of DNA encoding an L chain of a desired recombinant antibody or a fragment thereof and 2 copies of DNA encoding an H chain of the antibody or a fragment thereof, wherein the transformed cell produces the recombinant antibody or a fragment thereof.

The present inventors have found that by using the transformed cell introduced with a vector containing 4 copies of DNA encoding the L chain of the recombinant antibody and 2 copies of DNA encoding the H chain thereof, there is a significantly superior increase in the production of the desired recombinant antibody compared to when using
1) a transformed cell introduced with 2 copies of the L chain and 1 copy of the H chain,
2) a transformed cell introduced with 6 copies of the L chain and 3 copies of the H chain, or
3) a transformed cell introduced with 8 copies of the L chain and 4 copies of the H chain.

Accordingly, the production method of the present invention is characterized by using transformed cells introduced with a vector that contains 4 copies of DNA encoding the L chain of a recombinant antibody or a fragment thereof and 2 copies of DNA encoding the H chain of the recombinant antibody or a fragment thereof in the same vector.

In the production method of the present invention, an antibody or a fragment thereof is produced by constructing a recombinant vector containing 4 copies of DNA encoding an L chain of an antibody of interest or a fragment thereof and 2 copies of DNA encoding an H chain of the antibody or a fragment thereof, introducing the vector into a cell, and culturing the transformed cell introduced with the vector to produce the antibody of interest or a fragment thereof. The construction of the recombinant vector, the introduction of the vector into host cells, and the selection of the transformed cells introduced with the vector can be carried out using conventional means well-known to those skilled in the art. Culturing the transformed cells to produce the desired polypeptide can also be appropriately carried out by those skilled in the art.

The production method of the present invention may include selecting the transformed cells introduced with the aforementioned vector based on the productivity of the desired recombinant antibody or a fragment thereof to obtain (multiple types of) clonal cell lines with high production of the antibody or a fragment thereof, and then culturing each of the obtained clonal cell lines to produce the antibody or a fragment thereof. Here, the clonal cell line refers to those derived from a selected single cell. When using the product as an antibody pharmaceutical, the clonal cell line is further selected based on the quality of the product and the stability of the cells, and it becomes a seed culture strain suitable for production culture. The selected clonal cell line, namely the seed cell line, is expanded in culture, then aliquoted into typically hundreds of vials, and then cryopreserved for use as a cell bank in the production of antibody drug substances.

The production method of the present invention can be applied to antibodies against any antigen. Examples of the antibodies include, but are not limited to, recombinant antibodies such as an anti-CD137 antibody, an anti-latent TGF-β1 antibody, an anti-latent myostatin antibody, an anti-complement (C1s) antibody, an anti-IL-8 antibody, an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

The antibodies produced by the production method of the present invention include not only monoclonal antibodies derived from animals such as humans, mice, rats, hamsters, rabbits, sheep, camels, and monkeys, but also genetically modified recombinant antibodies such as chimeric antibodies, humanized antibodies, human antibodies, and low-molecular-weight antibodies.

Moreover, the immunoglobulin class of the antibodies is not particularly limited, and any class such as IgG1, IgG2, IgG3, IgG4, IgG, IgA, IgD, IgE, or IgM may be used, but when the antibodies are used as pharmaceuticals, IgG and IgM are preferred.

Furthermore, in the present invention, the desired recombinant antibody may be an antibody modified using antibody engineering techniques such as a switch antibody, a recycling antibody, or a sweeping antibody.

The DNA encoding the L chain of the antibody and the DNA encoding the H chain of the antibody can generally be prepared as follows. mRNA is extracted from hybridomas, cells, phages, ribosomes, or the like that have genes expressing antibodies. From this mRNA, cDNA is synthesized using a reverse transcription reaction with reverse transcriptase. PCR is performed using primers having base sequences complementary to the L chain gene or H chain gene and the cDNA to amplify the L chain gene or H chain gene, and each gene is obtained by ligating with a cloning plasmid.

The desired recombinant antibody in the present invention include not only whole antibodies but also antibody fragments such as Fab, F(ab')2, and Fv. Each gene fragment, which is the DNA encoding the fragment of the L chain of the antibody and the DNA encoding the fragment of the H chain of the antibody, can also be cloned using the same method as described above.

When using the product as an antibody pharmaceutical, it is possible to determine the amino acid sequence of the antibody chain based on the gene obtained by the above method or acquired by another method, and then make modifications that can improve efficacy, function, properties, or the like. Such modifications include those made using antibody engineering techniques mentioned in the present description.

Vectors are useful for maintaining DNA encoding the recombinant antibody or a fragment thereof within host cells or expressing the recombinant antibody or a fragment. In the present invention, there are no particular limitations on the host cells into which the vector is introduced, and examples of the host cells include E. coli and various animal cells.

When, for example, E. coli (such as JM109, DH5α, HB101, or XL1Blue) is used as a host, the vector, preferably contains an "ori" for amplification in E. coli to amplify and prepare the vector in large quantities, and also preferably contains a selectable marker gene for transformed E. coli (for example, a drug-resistance gene that can be identified by some drug, such as ampicillin, tetracycline, kanamycin, or chloramphenicol). Examples of known vectors include M13-series vectors, pUC-series vectors, pBR322, pBluescript, and pCR-Script. When cDNA subcloning or excision is intended, examples of the vectors include, in addition to the above vectors, pGEM-T, pDIRECT, and pT7. Many vectors derived from these are widely distributed, and can be used in the present invention.

In one aspect of the present invention, the vector is an expression vector intended for the expression of an antibody or a fragment thereof in animal cells. Examples of the expression vector that can be used include plasmid vectors, or non-plasmid vectors such as viral vectors, cosmid vectors, bacterial artificial chromosomes (BAC), and yeast artificial chromosomes (YAC). In a preferred aspect, the expression vector of the present invention is a plasmid vector designed for expression in animal cells. In the present invention, the expression vector is constructed to include 4 copies of DNA encoding the L chain of an antibody of interest or a fragment thereof and 2 copies of DNA encoding the H chain of the antibody or a fragment thereof, and is used to establish a cell line with high productivity of the antibody molecule or antibody fragment of interest.

Various gene expression vectors that function in mammalian cells are widely distributed, and necessary vectors can be selected according to your purpose. Alternatively, it is possible to use ones derived from known gene expression vectors with appropriate modifications in the structure. It is also possible to use a custom-designed vector which is created to ensure that the antibody molecule of interest is efficiently expressed in host cells. Examples of the basic components of the expression vectors include promoters and enhancers to drive expression of downstream genes, protein coding regions (ORFs) of genes of interest, polyA addition signals for transcription termination of upstream ORFs, marker genes for drug selection or visualization, and origins of replication (such as pUC ori for E. coli and SV40 ori for mammalian cells). Furthermore, to improve translation efficiency, a Kozak sequence may be placed before the start codon in the ORF.

In the construction of gene expression vectors, it is important to use strong promoters and enhancers that function efficiently within the target cells, for example, to establish stable high-expression cell lines. To construct an expression vector, the gene of interest is inserted downstream of promoters and enhancers. Examples of representative ones include the SV40 promoter, the MMLV-LTR promoter, the EF-1α promoter, the CMV promoter, the mouse β-globin promoter (mBGP), and the SRα promoter.

The CAG promoter is composed as a hybrid promoter by combining a CMV enhancer with a modified β-actin promoter derived from chicken (Niwa et al., Gene. (1991) 108, 193). The CAG promoter is a strong promoter that is not dependent on cell type, and expression vectors that transcribe the gene of interest using the CAG promoter can be expected to have higher expression of the target protein compared to expression vectors based on the CMV promoter. Modified promoters derived from the CAG promoter are also known (JP Patent No. 5670330 (WO2010/015079)). The eCAPE promoter (SEQ ID NO: 13), another modified CAG promoter, is an example of a preferred promoter available in the present invention for the expression of antibody polypeptide chains.

The expression vector preferably has a gene for the selection of transformed cells. Examples of such a gene include drug-resistance genes that can be identified by drugs (such as neomycin, G418, kanamycin, hygromycin, ampicillin, and puromycin), and fluorescent marker genes that can be sorted by cell sorting.

When obtaining high-expression cells through a gene amplification process that increases gene copy numbers in host cells, the expression vector may contain selection markers such as aminoglycoside transferase (APH) gene, thymidine kinase (TK) gene, Escherichia coli xanthine-guanine phosphoribosyl transferase (Ecogpt) gene, and dihydrofolate reductase (dhfr) gene.

It is known that mRNA with polyA is stabilized within cells, and it is preferable for the expression vector to have a necessary polyA signal to add polyA to the gene, such as mouse β globin polyA signal, bovine growth hormone polyA signal (bGHpA), and SV40 polyA signal.

Examples of the expression vector used to produce a desired antibody or a fragment thereof in the present invention include a plasmid vector that contains 4 copies of an L chain expression unit and 2 copies of an H chain expression unit. The expression unit is the unit of gene expression comprising a promoter, a gene coding sequence, and a terminator sequence (polyA signal). Typically, the L-chain expression unit is created by connecting a promoter upstream of an antibody L-chain gene and a polyA signal sequence downstream thereof. Similarly, the H chain expression unit is created by connecting a promoter upstream of an antibody H chain gene and a polyA signal sequence downstream thereof.

In the present invention, the cells used for producing or expressing an antibody or a fragment thereof are preferably animal cells. Examples of the known animal cells include mammalian cells such as CHO cells (J. Exp. Med. (1995) 108, 945), COS cells, 3T3 cells, myeloma cells, BHK (baby hamster kidney) cells, HeLa cells, Vero cells, amphibian cells such as African clawed frog oocytes (Valle, et al., Nature (1981) 291, 358-340), or insect cells such as Sf9, Sf21, and Tn5.

In animal cells, CHO cells are particularly preferred when the high-level expression is desired. As the CHO cells, particularly, dhfr-CHO (Proc. Natl. Acad. Sci. USA (1980) 77, 4216-4220), which are CHO cells lacking the dihydrofolate reductase (DHFR) gene, and CHO K-1 (Proc. Natl. Acad. Sci. USA (1968) 60, 1275) can be suitably used. Two cell lines, the DXB11 strain and DG44 strain, are widely distributed as CHO cells lacking dhfr.

The introduction of the vector into host cells can be performed by well-known methods such as the calcium phosphate method, the DEAE-dextran method, the method using cationic liposome DOTAP (manufactured by Boehringer Mannheim), the electroporation method, and lipofection.

The cells of the present invention may express an antibody or a fragment thereof in a transient expression system or in a stable expression system, but those that express an antibody or a fragment thereof in a stable expression system are preferable.

The transient expression system is a method in which a circular plasmid is taken up into cells and expressed using methods such as the calcium phosphate method, electroporation method, or lipofection method. The circular plasmid has a low efficiency of being inserted into chromosomes, and the target genes are often present outside the chromosomes. It is thus difficult to maintain long-term expression of the target gene from the circular plasmid.

The stable expression system is a method of introducing a linear plasmid prepared by a restriction enzyme treatment or the like into cells using methods such as the calcium phosphate method, the electroporation method, or the lipofection method, allowing the plasmid to be inserted into the chromosome, thereby enabling gene expression. It is thus possible to maintain the expression of the target gene for a long period. Linear plasmids have a higher efficiency of insertion into chromosomes than circular plasmids, and thus have higher efficiency of maintaining the target gene on the chromosome. In addition, the introduction of drug-resistance genes into the plasmid enables drug selection, allowing for the efficient selection of cells in which the target gene is maintained on the chromosome. Examples of animal cells used in stable expression systems include CHO cells, NS0 cells, and SP2/0 cells, with CHO cells being preferred.

In one aspect, the present invention is intended for the production of a specific antibody. Examples of the antibody of interest include the following antibodies.

The anti-CD137 antibody, for example, is the anti-human CD137 antibody described in WO2020/032230, and more specifically, the anti-CD137 agonist antibody called "STA551". STA551 is a switch antibody that binds to CD137 in an adenosine triphosphate (ATP)-dependent manner to exert agonist activity.

The anti-latent TGF-β1 antibody is a cross-species anti-latent TGF-β1 antibody described in, for example, WO2021/039945, and more specifically, it may be a monoclonal antibody called "SOF10" or "RG6440".

The anti-latent myostatin antibody can be, for example, a sweeping antibody called "GYM329" or "RG6237".

The anti-IL-8 antibody can be, for example, a recycling antibody called "AMY109".

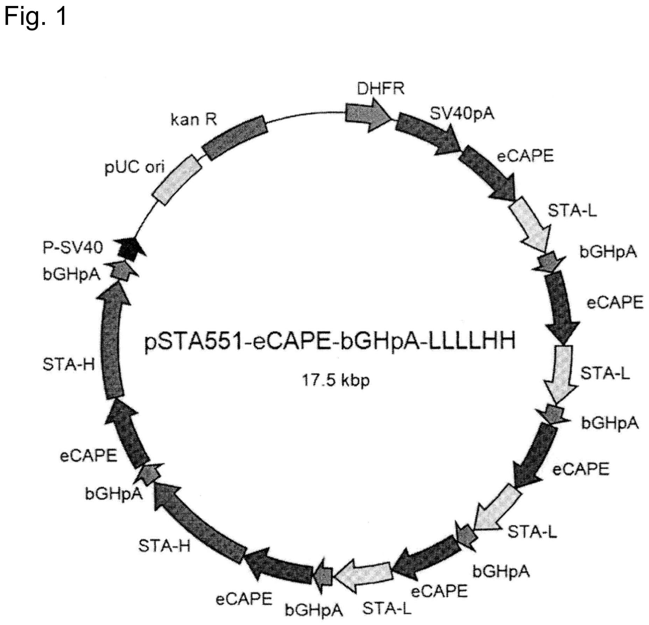

In a preferred aspect, the present invention may use a vector constructed for the purpose of expressing the antibody STA551. A specific example of the vector is pSTA551-eCAPE-bGHpA-LLLLHH (Fig. 1), which is introduced into dhfr gene-deficient CHO cells, the host cells for antibody production.

pSTA551-eCAPE-bGHpA-LLLLHH contains 4 copies of the L chain expression unit and 2 copies of the H chain expression unit of antibody STA551. In each expression unit, the eCAPE promoter is placed upstream of the STA551 H chain (SEQ ID NO: 11) or L chain (SEQ ID NO: 12), and bGHpA is placed downstream of the STA551 H chain or L chain as a polyA signal. This plasmid also contains a DHFR gene followed by SV40 polyA (SV40 pA). DHRF is designed to be expressed by a promoter in the chromosome when this plasmid is introduced into the chromosome of the cells, contributing to the selection of transformants. The plasmid also contains an SV40 promoter. This promoter, when introduced into the chromosome of the cells, expresses a hygromycin B resistance gene preinstalled on the chromosome, contributing to the selection of transformants. The plasmid also contains pUC ori and a kanamycin resistance gene (kan R). pUC ori functions as the origin of replication in E. coli during plasmid preparation. The kanamycin resistance gene contributes to the selection of E. coli that retains the plasmid.

In one aspect, the present invention is a CHO cell introduced with a vector containing 4 copies of the L-chain expression unit of an antibody and 2 copies of the H-chain expression unit of the antibody. In each expression unit, a CAG promoter or eCAPE promoter is placed upstream of the H-chain or L-chain. It is preferable that the antibody is one of STA551, SOF10, RAY121, AMY109, GYM329, or S12pre, more preferably STA551, SOF10, or RAY121, and most preferably STA551.

A polypeptide encoded by a gene of interest can be obtained by transforming cells with the gene of interest, and by culturing the transformed cells. In the present invention, an antibody or a fragment thereof is produced by culturing in vitro a cell introduced with a vector containing 4 copies of DNA encoding an L chain of the antibody or a fragment thereof and 2 copies of DNA encoding an H chain of the antibody or a fragment thereof.

In the present invention, culturing can be performed according to known methods. For culturing cells, the medium used for standard cell (preferably animal cell) culture can be used, and it is not particularly limited as long as it is the medium for animal cell culture. The medium usually contains amino acids, vitamins, lipid factors, energy sources, osmotic regulators, iron sources, and pH buffers. The medium may further contain, for example, trace metal elements, surfactants, proliferation auxiliary factors, nucleosides, or the like.

As the medium, it is also possible to use commercially available cell culture media such as D-MEM (Dulbecco's Modified Eagle Medium), D-MEM/F-12 1:1 Mixture (Dulbecco's Modified Eagle Medium: Nutrient Mixture F-12), MEM, RPMI1640, IMDM, CHO-S-SFM II (Invitrogen), CHO-SF (Sigma-Aldrich), EX-CELL 301 (JRH biosciences), CD-CHO (Invitrogen), IS CHO-V (Irvine Scientific), PF-ACF-CHO (Sigma-Aldrich), and Basal10 (Ajinomoto Co., Inc.). At that time, serum supplements such as fetal calf serum (FCS) can also be used. Alternatively, the medium may be a serum-free medium. When considering protein purification, it is desirable to culture cell lines in a serum-free medium.

When the host cells are CHO cells lacking the dhfr gene, hypoxanthine and thymidine are added to the medium. When the host cell is a proline-auxotrophic CHO cell line derived from the DXB11 or DG44 strain, it may be beneficial to add proline to the medium.

When the cells are CHO cells, culturing of CHO cells can be performed using methods known to those skilled in the art. For example, culturing of CHO cells can be generally performed in an atmosphere with a gaseous CO2 concentration of 0 to 40%, preferably 2 to 10% at temperature of 30 to 39°C, preferably around 37°C.

The culture period of suitable cells for producing the desired recombinant antibody or a fragment thereof is usually 1 day to 3 months, preferably 1 day to 2 months, and more preferably 1 day to 1 month.

Various types of cell culture equipment for animal cell culture can be used, and examples thereof include fermenter-type tank culture equipment, air-lift-type culture equipment, culture flask-type culture equipment, spinner flask-type culture equipment, microcarrier-type culture equipment, fluidized bed-type culture equipment, hollow fiber-type culture equipment, roller bottle-type culture equipment, and packed bed-type culture equipment.

Culturing can be performed using any of the methods such as batch culture, fed-batch culture, or continuous culture, but fed-batch culture or continuous culture is preferred, and fed-batch culture is more preferred.

A composition containing an antibody or a fragment thereof can be recovered from a cultured product obtained by a cell culture step for producing a recombinant antibody or a fragment thereof. Here, "recover" refers to recovering the supernatant (culture supernatant) containing the antibody or a fragment thereof from the cultured product or culture solution obtained through the cell culture step, or recovering the filtrate containing the antibody or a fragment thereof using a filter. The solution recovered in this manner can be adjusted into a composition containing an appropriate concentration of the antibody or a fragment thereof through a step of purification by affinity column chromatography described later or the like, and a step of concentration.

The antibody or a fragment thereof obtained by the production method of the present invention can be purified to homogeneity. Isolation and purification of the antibody or a fragment thereof can be performed by the conventional isolation and purification methods used for polypeptides. For example, the antibody can be separated and purified by appropriately selecting and combining chromatography columns for affinity chromatography or the like, filters, ultrafiltration, salting out, dialysis, SDS-polyacrylamide gel electrophoresis, isoelectric focusing electrophoresis, and the like (Antibodies : A Laboratory Manual. Ed Harlow and David Lane, Cold Spring Harbor Laboratory, 1988), but the isolation and purification methods are not limited to these. The concentration of the antibody obtained as described above can be measured by the absorbance measurement or enzyme-linked immunosorbent assay (ELISA).

Examples of the columns used for affinity chromatography include Protein A columns and Protein G columns. For example, representative Protein A affinity columns are Hyper D, POROS, Sepharose F. F. (Pharmacia), and the like.

Examples of chromatographic methods other than affinity chromatography include ion-exchange chromatography, hydrophobic interaction chromatography, gel filtration, reversed-phase chromatography, and adsorption chromatography (Strategies for Protein Purification and Characterization : A Laboratory Course Manual. Ed Daniel R. Marshak et al., Cold Spring Harbor Laboratory Press, 1996). These chromatographic methods can be performed using liquid chromatography such as HPLC and FPLC.

It is also possible to add desired modifications to the polypeptide or partially remove peptide segments by applying suitable polypeptide-modifying enzymes to the polypeptide before or after purification. Examples of the polypeptide-modifying enzymes include trypsin, chymotrypsin, lysyl endopeptidase, protein kinase, and glucosidase. It is also possible to generate modified antibodies by chemically modifying the obtained antibodies, for example, by conjugating them with various molecules such as polyethylene glycol (PEG).

When the antibody or a fragment thereof produced by the method of the present invention has biological activity that can be utilized as a pharmaceutical, the polypeptide can be mixed and formulated with a pharmaceutically acceptable carrier or additive to prepare the pharmaceutical.

Examples of the pharmaceutically acceptable carrier and additive include water, a pharmaceutically acceptable organic solvent, collagen, polyvinyl alcohol, polyvinylpyrrolidone, a carboxyvinyl polymer, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, vaseline, paraffin, stearyl alcohol, stearic acid, human serum albumin (HSA), mannitol, sorbitol, lactose, and a surfactant acceptable as a pharmaceutical additive.

The actual additives are selected alone or in appropriate combination from the above according to the dosage form of the therapeutic agent of the present invention, but the additives are not limited to these. For example, when used as an injectable formulation, the purified polypeptide can be dissolved in a solvent such as physiological saline, buffer solution, or glucose solution, and mixed with an anti-adsorption agent such as Tween 80, Tween 20, gelatin, human serum albumin or the like to prepare the formulation. Alternatively, it may be lyophilized to form a dosage form that is dissolved and reconstituted before use. Examples of excipients for lyophilization include sugar alcohols or saccharides such as mannitol and glucose.

The effective dosage of an antibody or a fragment thereof is appropriately selected based on the type of antibody or a fragment thereof, the type of disease to be treated or prevented, the age of the patient, the severity of the disease, and the like. For example, when the antibody is an anti-glypican-3 antibody and is used as an anti-cancer agent, the effective dosage of the anti-glypican-3 antibody is selected from a range of 0.001 mg to 1000 mg per kg of body weight per dose. Alternatively, the effective dosage can be selected from 0.01 to 100000 mg/body per patient. However, the effective dosage are not limited to these dosages.

The method of administering the antibody or a fragment thereof may be either oral administration or parenteral administration, but preferably parenteral administration. Specific examples include injection (for example, systemic or local administration by intravenous injection, intramuscular injection, intraperitoneal injection, subcutaneous injection, or the like), nasal administration, pulmonary administration, and transdermal administration.

### EXAMPLES

Hereinafter, the present invention will be specifically described below by way of examples. Note that these examples are for the purpose of explaining the present invention and do not limit the scope of the present invention.

In the examples, a plasmid was constructed using the humanized anti-CD137 antibody "STA551" H chain gene (SEQ ID NO: 11) and L chain gene (SEQ ID NO: 12).

### Example 1: Production of humanized anti-CD137 antibody (STA551) using H2L4 plasmid, H3L6 plasmid, and H4L8 plasmid

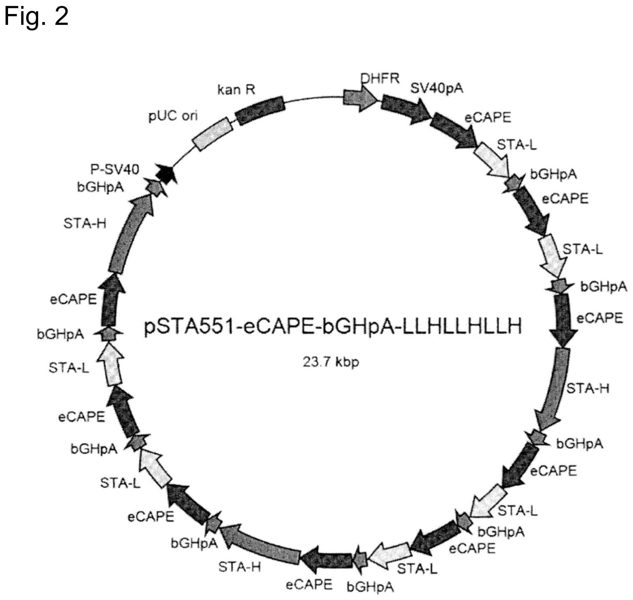

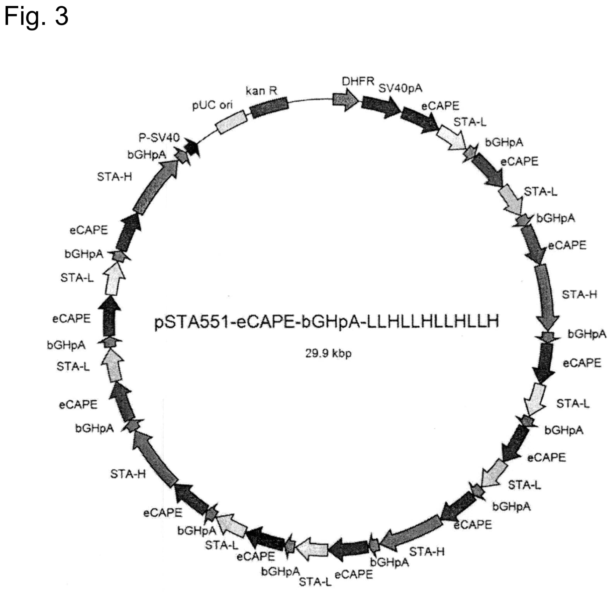

The eCAPE promoter (SEQ ID NO: 13) was connected upstream of each of the humanized anti-CD137 antibody H chain gene and the humanized anti-CD137 antibody L chain gene, and a bovine growth hormone (bgh) polyA signal sequence was further connected downstream thereof to create an H chain expression unit and an L chain expression unit. The H chain expression unit and the L chain expression unit were connected to a CHO cell introduction vector incorporated with a hygromycin resistance gene and a DHFR gene to prepare each of the H2L4 plasmid (Fig. 1) composed of 2 copies of the H chain and 4 copies of the L chain of the humanized anti-CD137 antibody gene, the H3L6 plasmid (Fig. 2) composed of 3 copies of the H chain and 6 copies of the L chain of the antibody gene, and the H4L8 plasmid (Fig. 3) composed of 4 copies of the H chain and 8 copies of the L chain of the antibody gene. Then, each plasmid was introduced into host cells derived from CHO cells DXB11 by electroporation. The cells were continuously cultured in the presence of 400 mg/ml of Hygromycin B to obtain cell lines introduced with the expression plasmid, and selection was performed based on antibody production.

For the selected 6 clonal cell lines introduced with the H2L4 plasmid, 9 clonal cell lines introduced with the H3L6 plasmid, and 6 clonal cell lines introduced with the H4L8 plasmid, a comparison of antibody production was performed by fed-batch culture using the cell culture equipment ambr15 (Sartorius AG). Basal 10 (Ajinomoto Co., Inc.) was used as the medium for the culture, with an initial culture solution volume of 10 mL, a culture temperature of 37°C, and a stirring speed of 800 rpm.

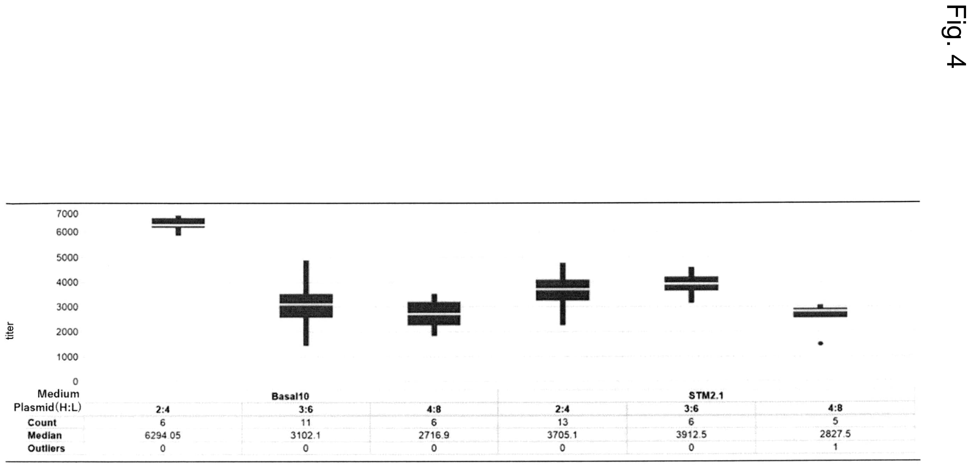

As a result of measuring the antibody concentration in the culture solution on day 14 after the start of culture, the 6 clonal cell lines introduced with the H2L4 plasmid had significantly higher antibody production than the other clones (3 results on the left in Fig. 4).

### Example 2: Culture evaluation with different media

Culture evaluation similar to that of Example 1 was performed with different media STM2.1 (Chugai proprietary chemically defined medium) on 13 clonal cell lines introduced with the H2L4 plasmid, 6 clonal cell lines introduced with the H3L6 plasmid, and 5 clonal cell lines introduced with the H4L8 plasmid. As a result, the clonal cell lines introduced with the H2L4 plasmid showed high antibody production even with different media (3 results on the right in Fig. 4).

### Example 3: Comparison with H1L2 plasmid

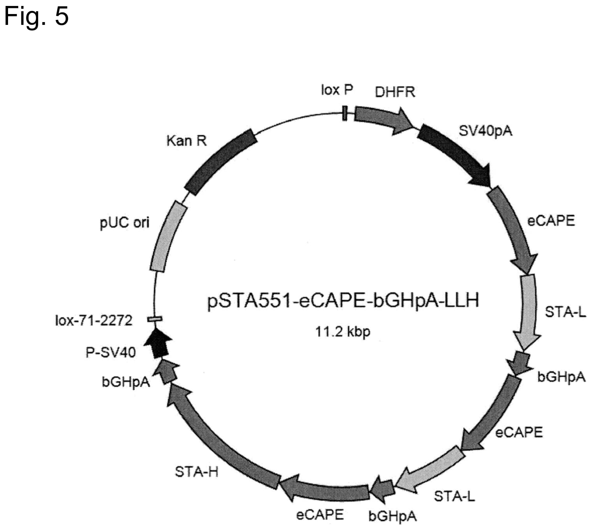

Furthermore, an H1L2 plasmid composed of 1 copy of the H chain and 2 copies of the L chain of the humanized anti-CD137 antibody gene (Fig. 5) was created as a comparative example and evaluated in the same manner as Examples 1 and 2.

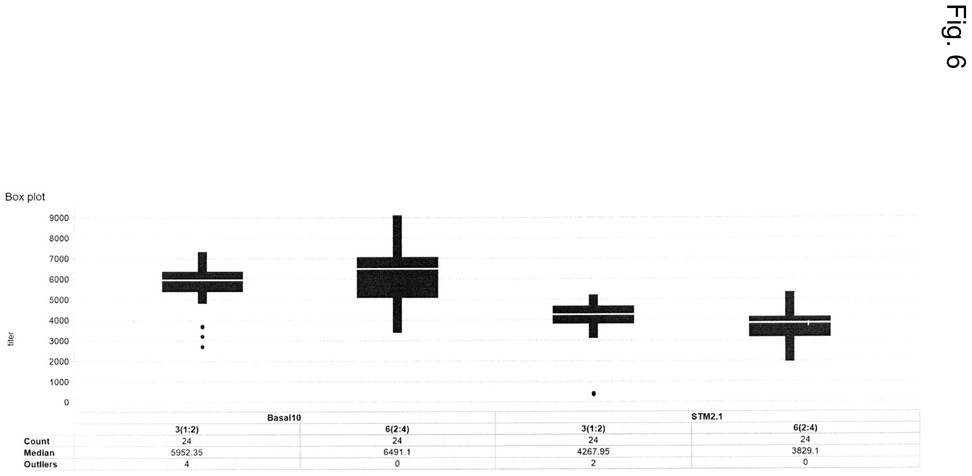

For the selected 24 clonal cell lines introduced with the H1L2 plasmid and 24 clonal cell lines introduced with the H2L4 plasmid, a fed-batch culture was performed using Basal 10 or STM2.1, and the antibody concentration in the culture solution was measured on the 14th day after the start of the culture. When using Basal 10, the clonal cell lines introduced with the H2L4 plasmid had significantly higher antibody production than the clonal cell lines introduced with the H1L2 plasmid (2 results on the left in Fig. 6).

From the above, it was confirmed that introducing 4 copies of the L chain and 2 copies of the H chain into host cells results in higher antibody production compared to when introducing with other copy numbers.

All publications, patents, and patent applications cited in the present description are incorporated herein by reference in their entirety.

### For sequences

STA551 is described in WO2020/032230 with the antibody name "A551-SCF057aPh/B379-Lamlib". In WO2020/032230, the structure and amino acid sequence of A551-SCF057aPh/B379-LamLib are described as shown below (see Table 51, Table 52, and the like). For convenience, each sequence is shown with SEQ ID described in WO2020/032230.
[VH] A551 (VH: SEQ 51, HCDR1: SEQ 7, HCDR2: SEQ 15, HCDR3: SEQ 20)
[CH] SCF057aPh (SEQ: 82)
[VL] B379 (VL: SEQ 60, LCDR1: SEQ 25, LCDR2: SEQ 26, LCDR3: SEQ 27)
[CL] Lamlib (SEQ: 63)

Namely, the H chain (A551-SCF057aPh) is a combination of the variable region A551 and the constant region SCF057aPh, and the L chain (B379-Lamlib) is a combination of the variable region B379 and the constant region Lamlib. For these amino acid sequences, SEQ ID NOs: 1 to 10 are respectively assigned in the sequence listing of the present application. For the nucleotide sequences of the H chain gene and L chain gene of the antibody STA551, SEQ ID NOs: 11 and 12 are respectively assigned in the sequence listing of the present application.

The sequences of the antibody STA551 (described as "A551-SCF057aPh/B379-LamLib" in WO2020/032230) (SEQ ID NOs: 1 to 12) and the sequence of eCAPE promoter (SEQ ID NO: 13) are shown below.

**Table 1: Amino acid sequence of H chain variable region**

| [VH] A551 (VH: SEQ 51, HCDR1: SEQ 7, HCDR2: SEQ 15, HCDR3: SEQ 20 as shown with the sequence numbers described in WO2020/032230) | | |
|---|---|---|
| [Table 1] | | |
| SEQ ID NO: 1 | <SEQ ID in WO2020/032230> | 51 ; <Sequence length> 122 |
| | Glu Val Gln Leu Val Glu Ser Gly Gly Gly Leu Val Lys Pro Gly Gly Ser Leu Arg Leu Ser Cys Ala Ala Ser Gly Phe Thr Phe Ser Thr Phe Thr Met Asn Trp Val Arg Gln Ala Pro Gly Lys Gly Leu Glu Trp Val Ser Ser Ile Ser Ser Lys Gly Ser Tyr Ile Glu Tyr Ala Glu Ser Phe Lys Val Arg Phe Thr Ile Ser Arg Asp Asn Ala Lys Asn Ser Leu Tyr Leu Gln Met Asn Ser Leu Arg Ala Glu Asp Thr Ala Val Tyr Tyr Cys Ala Arg Tyr Gly Ile Lys Asn Glu Leu Asn Trp Val Phe Asp Tyr Trp Gly Gln Gly Thr Leu Val Thr Val Ser Ser | |
| SEQ ID NO: 2 | <SEQ ID in WO2020/032230> | 7 ; <Sequence length> 5 |
| | Thr Phe Thr Met Asn | |
| SEQ ID NO: 3 | <SEQ ID in WO2020/032230> | 15 ; <Sequence length> 17 |
| | Ser Ile Ser Ser Lys Gly Ser Tyr Ile Glu Tyr Ala Glu Ser Phe Lys Val | |
| SEQ ID NO: 4 | <SEQ ID in WO2020/032230> | 20 ; <Sequence length> 13 |
| | Tyr Gly Ile Lys Asn Glu Leu Asn Trp Val Phe Asp Tyr | |

**Table 2: Amino acid sequence of H chain constant region**

| [CH] SCF057aPh (SEQ: 82 as shown with the sequence number described WO2020/032230) in | | |
|---|---|---|
| [Table 2] | | |
| SEQ ID NO: 5 | <SEQ ID in WO2020/032230> | 82 ; <Sequence length> 328 |
| | Ala Ser Thr Lys Gly Pro Ser Val Phe Pro Leu Ala Pro Ser Ser Lys Ser Thr Ser Gly Gly Thr Ala Ala Leu Gly Cys Leu Val Lys Asp Tyr Phe Pro Glu Pro Val Thr Val Ser Trp Asn Ser Gly Ala Leu Thr Ser Gly Val His Thr Phe Pro Ala Val Leu Gln Ser Ser Gly Leu Tyr Ser Leu Ser Ser Val Val Thr Val Pro Ser Ser Ser Leu Gly Thr Gln Thr Tyr Ile Cys Asn Val Asn His Lys Pro Ser Asn Thr Lys Val Asp Lys Arg Val Glu Pro Lys Ser Cys Asp Lys Thr His Thr Cys Pro Pro Cys Pro Ala Pro Glu Leu Trp Asn Gly Pro Ser Val Phe Leu Phe Pro Pro Lys Pro Lys Asp Thr Leu Met Ile Ser Arg Thr Pro Glu Val Thr Cys Val Val Val Asp Val Ser Asp Glu Asp Pro Glu Val Lys Phe Asn Trp Tyr Val Asp Gly Val Glu Val His Asn Ala Lys Thr Lys Pro Arg Glu Glu Gln Tyr Asn Ser Thr Tyr Arg Val Val Ser Val Leu Thr Val Leu His Arg Asp Trp Leu Asn Gly Lys Glu Tyr Lys Cys Lys Val Ser Asn Lys Ala Leu Pro Lys Pro Ile Glu Lys Thr Ile Ser Lys Ala Lys Gly Gln Arg Arg Glu Pro Gln Val Tyr Thr Leu Pro Pro Ser Arg Glu Glu Met Thr Lys Asn Gln Val Ser Leu Thr Cys Leu Val Lys Gly Phe Tyr Pro Ser Asp Ile Ala Val Glu Trp Glu Ser Asn Gly Gln Pro Glu Asn Asn Tyr Lys Thr Thr Pro Pro Val Leu Asp Ser Asp Gly Ser Phe Phe Leu Tyr Ser Lys Leu Thr Val Asp Lys Ser Arg Trp Gln Gln Gly Asn Val Phe Ser Cys Ser Val Met His Glu Ala Leu His Asn His Tyr Thr Gln Lys Ser Leu Ser Leu Ser Pro | |

**Table 3: Amino acid sequence of L chain variable region**

| [VL] B379 (VL: SEQ 60, LCDR1: SEQ 25, LCDR2: SEQ 26, LCDR3: SEQ 27 as shown with the sequence numbers described in WO2020/032230) | | |
|---|---|---|
| Table 3] | | |
| SEQ ID NO: 6 | <SEQ ID in WO2020/032230> | 60 ; <Sequence length> 110 |
| | Gln Ser Ala Leu Thr Gln Pro Pro Ser Ala Ser Gly Ser Pro Gly Gln Thr Val Thr Ile Ser Cys Thr Gly Thr Ser Thr Asp Val Gly Tyr Tyr Glu Tyr Val Ser Trp Tyr Gln Gln His Pro Gly Lys Ala Pro Lys Leu Met Ile Tyr Glu Thr Ser Lys Arg Leu Ser Gly Val Pro Asp Arg Phe Ser Gly Ser Lys Ser Gly Asn Thr Ala Ser Leu Thr Val Ser Gly Leu Gln Ala Glu Asp Glu Ala Asp Tyr Tyr Cys Ser Ser Tyr Arg Tyr Glu His Gln Val Ser Phe Gly Gly Gly Thr Lys Leu Thr Val Leu | |
| SEQ ID NO: 7 | <SEQ ID in WO2020/032230> | 25 ; <Sequence length> 14 |
| | Thr Gly Thr Ser Thr Asp Val Gly Tyr Tyr Glu Tyr Val Ser | |
| SEQ ID NO: 8 | <SEQ ID in WO2020/032230> | 26 ; <Sequence length> 7 |
| | Glu Thr Ser Lys Arg Leu Ser | |
| SEQ ID NO: 9 | <SEQ ID in WO2020/032230> | 27 ; <Sequence length> 10 |
| | Ser Ser Tyr Arg Tyr Glu His Gln Val Ser | |

**Table 4: Amino acid sequence of L chain constant region**

| [CL] Lamlib (SEQ: 63 as shown with the sequence number described in WO2020/032230) | | |
|---|---|---|
| [Table 4] | | |
| SEQ ID NO: 10 | <SEQ ID in WO2020/032230> | 63 ; <Sequence length> 106 |
| | Gly Gln Pro Lys Ala Ala Pro Ser Val Thr Leu Phe Pro Pro Ser Ser Glu Glu Leu Gln Ala Asn Lys Ala Thr Leu Val Cys Leu Ile Ser Asp Phe Tyr Pro Gly Ala Val Thr Val Ala Trp Lys Ala Asp Ser Ser Pro Val Lys Ala Gly Val Glu Thr Thr Thr Pro Ser Lys Gln Ser Asn Asn Lys Tyr Ala Ala Ser Ser Tyr Leu Ser Leu Thr Pro Glu Gln Trp Lys Ser His Arg Ser Tyr Ser Cys Gln Val Thr His Glu Gly Ser Thr Val Glu Lys Thr Val Ala Pro Thr Glu Cys Ser | |

### DNA sequences of H chain gene and L chain gene of "STA551"

>STA551 H (SEQ ID NO: 11) 
ATGGAGctgGGGCTGcgcTGGGTTTTCCTCGTTGCTATAttagaaGGTGTCCAGTGTgaa
GTGCAACTGGTGGAGTCTGGGGGAGGCTTGGTCAAACCTGGGGGGTCCCTGAGA
CTCTCC
TGTGCTGCCTCTGGATTCACCTTCTCTacctttACCATGAATTGGGTCCGCCAAGCTCC
C
GGCAAGGGGCTGGAATGGGTGTCATCCATTTCatcaaaAgggTCtTATATTgagTATGCT
GAATCCttcaaagtcCGATTCACCATCTCAAGAGACAACGCCAAGAACTCTCTGTATCT
G
CAAATGAACTCCCTGAGAGCCGAGGACACCGCCGTGTATTACTGTGCCAGATATG
GTatc
aaaaatgagctgaatTGGGTTTTGACTACTGGGGCCAAGGCACCCTGGTGACAGTGTCC
TCTGCCTCCACAAAaGGCCCATCCGTGTTCCCCCTGGCTCCTTCCTCTAAGTCTAC
CTCC
GGCGGCACAGCCGCTCTGGGATGTCTGGTGAAGGATTATTTCCCTGAACCCGTGA
CCGTG
 
TCTTGGAACTCCGGCGCCCTGACCTCCGGCGTGCACACATTTCCCGCCGTGCTAC
AGTCC
TCTGGCCTGTACTCTCTGTCCTCTGTGGTGACAGTGCCCTCCTCTTCCCTGGGCAC
CCAG
ACATATATCTGTAATGTTAATCACAAGCCCTCCAATACAAAAGTGGACAAGAGAGT
GGAG
CCTAAGTCTTGTGATAAGACCCATACATGCCCCCCTTGTCCTGCCCCTGAACTGtgga
ac
GGCCCTTCAGTGTTCCTGTTTCCACCCAAACCAAAGGACaccCTGATGATCTCAAG
GACC
CCTGAGGTGACATGCGTGGTGGTGGACGTGTCTgacGAGgacCCCGAAGTGAAGTTT
AAT
TGGTACGTGGATGGCGTGGAAGTGCATAATGCCAAGACAAAGCCCAGAGAGGAG
CAGTAC
AACtcaACaTATAGGGTGGTGTCTGTGCTGaccGTGCTGCACCgGGACTGGCTGAACG
GC
AAGGAGTATAAGTGCAAGGTGTCCAATaaaGCCTTGCCTaagCCCATCGAGaaaACCAT
C
TCTAAGGCTAAGGGACAGcggCGGGAGCCTCAAGTGTACACACTGCCTCCATCCAG
AGAG
GAGATGACCAAGAACCAAGTGTCTCTGACATGTCTGGTCAAGGGCTTCTATCCAT
CTGAC
ATCGCTGTGGAGTGGGAGTCTAATGGCCAGCCCGAGAACAATTACAAGACCACAC
CCCCT
GTGCTGGACTCTGATGGCTCCTTCTTTCTGTATTCCAAACTGACCGTGGATAAGTC
TAGG
TGGCAGCAGGGCAACGTGTTCTCCTGTTCTGTCATGCACGAGGCACTGCACAACC
ATTAC
 ACCCAGaagTCACTGTCACTGTCACCTTGA
>STA551 L (SEQ ID NO: 12) 
ATGGCCTGGtcCcctCTGCTCCTCACCCTCCTCgctcactgcACAGGGTCCTGGGCCCAA
TCCGCACTGACTCAACCTCCATCCGCCTCTGGTTCCCCTGGACAGACAGTTACCAT
CTCC
TGTacTGGCaccTCAACTGATGTGGGTtAtTACgaATATGTATCCTGGTATCAACAGCAC
CCTGGAAAGGCTCCTAAACTGATGATTTATgagACTTCCAAAcgtctgTCTGGTGTCCC
T
GATCGTTTTTCCGGCTCCAAATCCGGCAACACTGCCTCCCTGACTGTTTCCGGTTT
GCAA
GCTGAGGACGAGGCAGACTATtacTGCTCATCcTATcgttatgaAcatcaggttTCcTTC
GGCGGAGGGACCAAACTGACCGTCCTAGGTCAGCCCAAGGCTGCCCCCTCTGTC
ACTCTG
TTCCCACCCTCCTCTGAGGAGCTTCAAGCCAACAAGGCCACACTCGTATGTCTCAT
ATCT
GACTTCTACCCTGGCGCCGTGACAGTGGCCTGGAAGGCAGATTCCTCACCCGTCA
AGGCT
GGCGTGGAaACCACCACACCCTCCAAACAATCTAACAACAAATACGCTGCCTCAT
CCTAT
CTGTCCCTGACTCCTGAGCAGTGGAAGTCCCACAGATCCTACTCCTGCCAAGTCA
CACAT
GAAGGGTCCACCGTGGAGAAGACAGTGGCCCCTACAGAATGTTCCTGA
DNA sequence of eCAPE promoter (SEQ ID NO: 13)

GTCGACATTGATTATTGACTAGTTATTAATAGTAATCAATTACGGGGTCATTAGTTCA
TA
GCCCATATATGGAGTTCCGCGTTACATAACTTACGGTAAATGGCCCGCCTGGCTGA

CCGC
CCAACGACCCCCGCCCATTGACGTCAATAATGACGTATGTTCCCATAGTAACGCCA
ATAG
GGACTTTCCATTGACGTCAATGGGTGGAGTATTTACGGTAAACTGCCCACTTGGCA
GTAC
ATCAAGTGTATCATATGCCAAGTACGCCCCCTATTGACGTCAATGACGGTAAATGG
CCCG
CCTGGCATTATGCCCAGTACATGACCTTATGGGACTTTCCTACTTGGCAGTACATCT
ACG
TATTAGTCATCGCTATTACCATGGTCGAGGTGAGCCCCACGTTCTGCTTCACTCTCC
CCA
TCTCCCCCCCCTCCCCACCCCCAATTTTGTATTTATTTATTTTTTAATAGCCAATCAG
AG
CGGCGCGCTCCGAAAGTTTCCTTTTATGGCGAGGCGGCGGCGGCGGCCCTAT
AAAAA
GCGAAGCGCGCGGCGGGCGGGAGTCGCTGCGCGCTGCCTTCGCCCCGTGCCCCG
CTCCGC
CGCCGCCTCGCGCCGCCCGCCCCGGCTCTGACTGACCGCGTTACTCCCACAGGTG
AGCGG
GCGGGACGGCCCTTCTCCTCCGGGCTGTAATTAGCGGGGGGACGGCTGCCTTCGG
GGGGG
ACGGGGCAGGGCGGGGTTCGGCTTCTGGCGTGTGACCGGCGGCTCTAGAGCCTC
TGCTAA
CCATGTTCATGCCTTCTTCTTTTTCCTACAGCTCCTGGGCAACGTGCTGGTTATTGT
GCT
GTCTCATCATTTTGGCAAAccggt

### INDUSTRIAL APPLICABILITY

The present invention can be used for the production of antibodies. The present invention can be applied to any antibody-producing cell.

SEQUENCE LISTING

## Claims

1. A method for producing an antibody or a fragment thereof, the method comprising using a cell introduced with a vector containing 4 copies of DNA encoding an L chain of an antibody or a fragment thereof and 2 copies of DNA encoding an H chain of the antibody or a fragment thereof.

2. The method according to claim 1, wherein the cell is an animal cell.

3. The method according to claim 2, wherein the animal cell is a Chinese hamster ovary cell.

4. The method according to any one of claims 1 to 3, wherein the antibody is a chimeric antibody, a humanized antibody, or a human antibody.

5. The method according to any one of claims 1 to 3, wherein the antibody is a switch antibody, a recycling antibody, or a sweeping antibody.

6. The method according to any one of claims 1 to 3, wherein the antibody is selected from the group consisting of an anti-CD137 antibody, an anti-latent TGF-β1 antibody, an anti-latent myostatin antibody, an anti-complement (C1s) antibody, an anti-IL-8 antibody, an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

7. The method according to claim 6, wherein the antibody is an anti-CD137 agonist switch antibody STA551, an anti-latent TGF-β1 monoclonal antibody SOF10/RG6440, an anti-latent myostatin sweeping antibody GYM329/RG6237, an anti-complement (C1s) antibody RAY121, an antibody S12pre, or an anti-IL-8 recycling antibody AMY109.

8. The method according to claim 6, wherein the antibody is an anti-CD137 antibody STA551.

9. The method according to any one of claims 1 to 3, wherein the cell stably expresses the antibody or a fragment thereof.

10. A recombinant vector comprising 4 copies of DNA encoding an L chain of an antibody or a fragment thereof and 2 copies of DNA encoding an H chain of the antibody or a fragment thereof.

11. A cell introduced with the vector according to claim 10.

12. The cell according to claim 11, which stably expresses the antibody or a fragment thereof.

13. The vector according to claim 10 or the cell according to claim 11 or 12, wherein the antibody is selected from the group consisting of an anti-CD137 antibody, an anti-latent TGF-β1 antibody, an anti-latent myostatin antibody, an anti-complement (C1s) antibody, an anti-IL-8 antibody, an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

14. The vector or cell according to claim 13, wherein the antibody is an anti-CD137 agonist switch antibody STA551, an anti-latent TGF-β1 monoclonal antibody SOF10/RG6440, an anti-latent myostatin sweeping antibody GYM329/RG6237, an anti-complement (C1s) antibody RAY121, an antibody S12pre, or an anti-IL-8 recycling antibody AMY 109.

15. The vector or cell according to claim 13, wherein the antibody is an anti-CD137 antibody STA551.

16. A method for producing a pharmaceutical containing an antibody or a fragment thereof, wherein the antibody or a fragment thereof is produced by the method according to any one of claims 1 to 9, and the obtained antibody is mixed and formulated with a pharmaceutically acceptable carrier or additive to prepare the pharmaceutical.
